(19) Europäisches Patentamt
European Patent Office
Office européen des brevets

(11) EP 4 484 007 A1

(12) EUROPEAN PATENT APPLICATION
published in accordance with Art. 153(4) EPC

(43) Date of publication:
**01.01.2025 Bulletin 2025/01**

(21) Application number: **23760049.9**

(22) Date of filing: **22.02.2023**

(51) International Patent Classification (IPC):
***B01J 31/24*** (2006.01) ***C07B 61/00*** (2006.01) ***C07C 51/00*** (2006.01) ***C07C 51/02*** (2006.01) ***C07C 53/06*** (2006.01)

(52) Cooperative Patent Classification (CPC):
**B01J 31/24; C07B 61/00; C07C 51/00; C07C 51/02; C07C 53/06**

(86) International application number:
**PCT/JP2023/006496**

(87) International publication number:
**WO 2023/163050 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.02.2022 JP 2022028470**

(71) Applicant: **NITTO DENKO CORPORATION
Ibaraki-shi
Osaka 567-8680 (JP)**

(72) Inventors:
- **HIRANO, Makoto
Ibaraki-shi, Osaka 567-8680 (JP)**
- **MATSUDA, Hirokazu
Ibaraki-shi, Osaka 567-8680 (JP)**
- **SERIU, Masaya
Ibaraki-shi, Osaka 567-8680 (JP)**
- **PIDKO, Evgeny Alexandrovich
3584 HT Utrecht (NL)**
- **FILONENKO, Georgy Alexandrovich
2613 VG Delft (NL)**
- **REBREYEND, Christophe
2624 BC Delft (NL)**

(74) Representative: **Hoffmann Eitle
Patent- und Rechtsanwälte PartmbB
Arabellastraße 30
81925 München (DE)**

# (54) CATALYST REACTION METHOD, FORMATE PRODUCTION METHOD, AND FORMIC ACID PRODUCTION METHOD

(57) The present invention relates to a catalyst reaction method in which in a catalyst reaction using at least one catalyst selected from the group consisting of a metal complex represented by the general formula (1A) described in the specification, a tautomer or stereoisomer of the metal complex, and a salt of the metal complex or the tautomer or stereoisomer, a ligand represented by the general formula (1B) described in the specification is used in such a manner that a ratio of a substance amount of the ligand to a substance amount of the catalyst in a reaction system is greater than 0 to 15.

EP 4 484 007 A1

## Description

### TECHNICAL FIELD

The present invention relates to a catalyst reaction method, a method for producing a formate, and a method for producing formic acid.

### BACKGROUND ART

Various transition metal complexes composed of transition metals and ligands are used as catalysts in organic synthesis reactions.

In view of the problems of global warming, depletion of fossil fuel, and the like, high expectations are placed on hydrogen energy as next-generation energy, and a method for producing formic acid from carbon dioxide ($CO_2$) and hydrogen ($H_2$) in the presence of a catalyst is investigated.

Patent Literature 1 describes a method for producing formic acid by subjecting carbon dioxide under the reaction of carbon dioxide with hydrogen in the presence of a catalyst containing an element from Groups 8, 9, or 10 of the Periodic System, a tertiary amine (I), and a polar solvent.

Patent Literature 2 investigates a hydroformylation process using a catalyst, and describes that supplementary or additional ligands can be supplied to a reaction medium of the hydroformylation process.

### CITATION LIST

### PATENT LITERATURE

Patent Literature 1: JP5734286B
Patent Literature 2: JP2013-513063A

### SUMMARY OF INVENTION

### TECHNICAL PROBLEM

However, in the related art, even if a catalyst has an excellent catalyst efficiency at the start of a reaction, catalyst activity decreases as the catalyst reaction progresses, resulting in a decrease in the catalyst efficiency.

Therefore, the present invention relates to a catalyst reaction method, a method for producing a formate, and a method for producing formic acid that can improve catalyst efficiency.

### SOLUTION TO PROBLEM

As a result of intensive investigations, the present inventors have found that catalyst efficiency can be improved by using a catalyst having a specific metal complex structure and a ligand in a specific ratio, and have completed the invention.

Means for solving the above problems are as follows.

<1>
A catalyst reaction method, in which
in a catalyst reaction using at least one catalyst selected from the group consisting of a metal complex represented by the following general formula (1A), a tautomer or stereoisomer of the metal complex, and a salt of the metal complex or the tautomer or stereoisomer, a ligand represented by the following general formula (1B) is used in such a manner that a ratio of a substance amount of the ligand to a substance amount of the catalyst in a reaction system is greater than 0 to 15.

[Chem. 1]

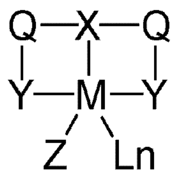

(1A)

(In the general formula (1A), X represents a zero-valent to monovalent atomic group containing a typical element of Groups 13 to 15 and capable of coordinating to M, Q each independently represents a crosslinked structure containing a typical element of Groups 14 to 16 and connecting Y and X, Y each independently represents a zero-valent to monovalent atomic group containing a typical element of Groups 14 to 16 and capable of coordinating to M, M represents a metal atom, Z represents a halogen atom or a hydrogen atom, n represents an integer of 0 to 3, and when more than one L are present, L each independently represents a neutral or anionic ligand.)

[Chem. 2]

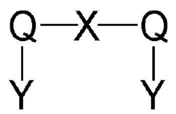

(1B)

(In the general formula (1B), X represents the zero-valent to monovalent atomic group containing the typical element of Groups 13 to 15 and capable of coordinating to M in the general formula (1A), Q each independently represents a crosslinked structure containing a typical element of Groups 14 to 16 and connecting Y and X, and Y each independently represents the zero-valent to monovalent atomic group containing the typical element of Groups 14 to 16 and capable of coordinating to M in the general formula (1A).)

<2>

The catalyst reaction method described in <1>, in which

the metal complex represented by the general formula (1A) is a metal complex represented by the following general formula (2A).

[Chem. 3]

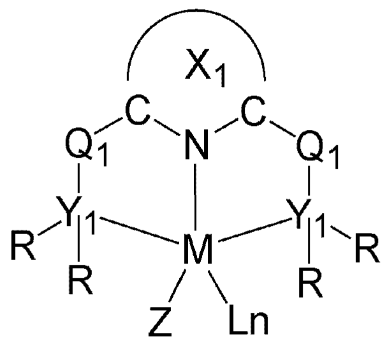

(2A)

(In the general formula (2A), $X_1$ represents a heteroaromatic ring formed with two carbon atoms and a nitrogen atom, which may have a substituent or may be combined with another substituent to form a ring, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

<3>

The catalyst reaction method described in <2>, in which

the metal complex represented by the general formula (2A) is a metal complex represented by the following general

formula (3A).

[Chem. 4]

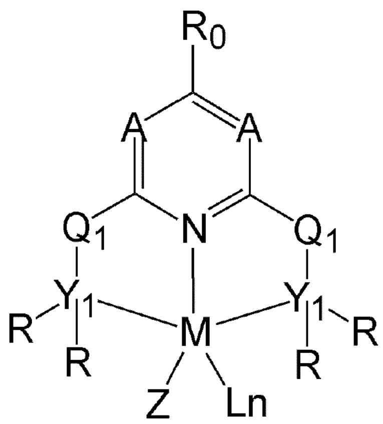

(3A)

(In the general formula (3A), $R_0$ represents a hydrogen atom or an alkyl group, A each independently represents CH, $CR_5$, or N, $R_5$ represents an alkyl group, an aryl group, an aralkyl group, an amino group, a hydroxy group, or an alkoxy group, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

<4>

The catalyst reaction method described in <1>, in which the metal atom represented by M is ruthenium.

<5>

The catalyst reaction method described in <1>, in which
the catalyst reaction is an oxidation reaction or a reduction reaction of an organic compound.

<6>

The catalyst reaction method described in <1>, in which
the catalyst reaction is a formate forming reaction.

<7>

The catalyst reaction method described in <6>, in which

the formate forming reaction is a method for producing a formate by allowing hydrogen to react with at least one compound selected from the group consisting of carbon dioxide, a hydrogen carbonate, and a carbonate in the presence of a solvent, and
the formate forming reaction is a two-phase system reaction in which an organic solvent and an aqueous solvent are present in a separated state in the solvent.

<8>

A method for producing a formate, the method including:
a first step of producing the formate by the catalyst reaction method described in <6> or <7>.

<9> A method for producing formic acid, the method including:

a first step of producing a formate by the catalyst reaction method described in <6> or <7>; and
a second step of protonating at least a part of the formate to form formic acid.

ADVANTAGEOUS EFFECTS OF INVENTION

The present invention can provide a catalyst reaction method, a method for producing a formate, and a method for producing formic acid that can improve catalyst efficiency.

DESCRIPTION OF EMBODIMENTS

Hereinafter, embodiments of the present invention will be described in detail. The present invention is not limited to the embodiments to be described below.

In the present specification, when the term "may have a substituent" is used, a type of the substituent, a position of the substituent, and the number of substituents are not particularly limited. The number of substituents may be, for example, one, two, three, or more. The substituent includes, for example, a monovalent non-metal atomic group excluding a hydrogen atom, and can be selected from the following substituent group A, for example.

(Substituent Group A)

The substituent group A includes an alkyl group, an aryl group, a heteroaryl group, an alkenyl group, an alkoxy group, an aryloxy group, a trialkylsilyl group, a dialkylarylsilyl group, a triarylsilyl group, a dialkylamino group, an amino group, a hydroxy group, and combinations thereof.

The alkyl group in the substituent group A includes a straight chain, branched or cyclic substituted or unsubstituted alkyl group. The alkyl group preferably includes an alkyl group having 1 to 30 carbon atoms, such as a methyl group, an ethyl group, an n-propyl group, an i-propyl group, a t-butyl group, an n-octyl group, an eicosyl group, or a 2-ethylhexyl group. From the standpoint of catalyst activity, an alkyl group having 12 or less carbon atoms is preferred, and a methyl group, an ethyl group, or a t-butyl group is preferred.

The aryl group in the substituent group A includes a substituted or unsubstituted aryl group having 6 to 30 carbon atoms, such as a phenyl group, a p-tolyl group, a naphthyl group, an m-chlorophenyl group, or an o-hexadecanoylaminophenyl group. An aryl group having 12 or less carbon atoms is preferred, and a phenyl group is more preferred.

The heteroaryl group in the substituent group A includes a substituted or unsubstituted heteroaryl group having 4 to 30 carbon atoms, such as a pyridyl group, a pyrrolidyl group, an imidazolyl group, an oxazolyl group, and a thiazolyl group.

The alkenyl group in the substituent group A includes a straight chain, branched or cyclic substituted or unsubstituted alkenyl group, and preferably an alkenyl group having 2 to 30 carbon atoms, such as a vinyl group, an n-propenyl group, an i-propenyl group, a t-butenyl group, an n-octenyl group. An alkenyl group having 6 or less carbon atoms is preferred.

The alkoxy group in the substituent group A includes a straight chain, branched or cyclic substituted or unsubstituted alkoxy group, and preferably a substituted or unsubstituted alkoxy group having 1 to 30 carbon atoms, such as a methoxy group, an ethoxy group, an isopropoxy group, a t-butoxy group, an n-octyloxy group, and a 2-methoxyethoxy group.

Embodiments of the invention are described in detail below.

A catalyst reaction method according to the embodiment of the invention, in which in a catalyst reaction using at least one catalyst selected from the group consisting of a metal complex represented by the following general formula (1A), a tautomer or stereoisomer of the metal complex, and a salt of the metal complex or the tautomer or stereoisomer, a ligand represented by the following general formula (1B) is used in such a manner that a ratio of a substance amount of the ligand to a substance amount of the catalyst in a reaction system is greater than 0 to 15.

[Chem. 5]

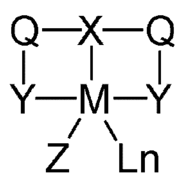

(1A)

(In the general formula (1A), X represents a zero-valent to monovalent atomic group containing a typical element of Groups 13 to 15 and capable of coordinating to M, Q each independently represents a crosslinked structure containing a typical element of Groups 14 to 16 and connecting Y and X, Y each independently represents a zero-valent to monovalent atomic group containing a typical element of Groups 14 to 16 and capable of coordinating to M, M represents a metal atom, Z represents a halogen atom or a hydrogen atom, n represents an integer of 0 to 3, and when more than one L are present, L each independently represents a neutral or anionic ligand.)

[Chem. 6]

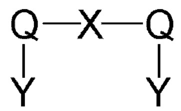

(1B)

(In the general formula (1B), X represents the zero-valent to monovalent atomic group containing the typical element of Groups 13 to 15 and capable of coordinating to M in the general formula (1A), Q each independently represents a crosslinked structure containing a typical element of Groups 14 to 16 and connecting Y and X, and Y each independently represents the zero-valent to monovalent atomic group containing the typical element of Groups 14 to 16 and capable of coordinating to M in the general formula (1A).)

A method for producing a formate according to another embodiment of the invention includes a first step of producing the formate by the catalyst reaction method.

A method for producing formic acid according to another embodiment of the invention includes a first step of producing a formate by the catalyst reaction method, and
a second step of protonating at least a part of the formate to form formic acid.

[Catalyst Reaction Method]

In the catalyst reaction method according to the embodiment of the invention, in the catalyst reaction using at least one catalyst (hereinafter, also simply referred to as the "catalyst") selected from the group consisting of the metal complex represented by the above general formula (1A), a tautomer or stereoisomer of the metal complex, and a salt of the metal complex or the tautomer or stereoisomer, a ligand represented by the above general formula (1B) (hereinafter, also simply referred to as the "ligand") is used in such a manner that a ratio of a substance amount of the ligand to a substance amount of the catalyst in a reaction system is greater than 0 to 15.

By setting the ratio of the substance amount of the ligand to that of the catalyst (ligand/catalyst) within a specific range, deterioration of the catalyst due to oxygen, impurities, and the like in the reaction system during the progress of the catalyst reaction can be prevented, and an effect of improving the catalyst efficiency is achieved.

The ratio of the substance amount of the ligand to that of the catalyst is preferably 1 or more, more preferably 2 or more, and preferably 13 or less, and more preferably 12 or less.

By adding an excess of ligand to form a complex in the reaction system, even when the ligand coordinated to a metal in the catalyst is oxidized and deteriorated by oxygen and impurities included in the system, the deteriorated ligand and the added ligand are exchanged to restore a catalyst function, and therefore, stability of the catalyst can be improved.

Addition of the ligand into the reaction mixture may be carried out when the reaction mixture is prepared or may be carried out in the middle of the reaction. However, from the standpoint of process management, the addition is preferably carried out when the reaction mixture is prepared.

In the catalyst reaction method according to the embodiment of the present invention, the metal complex represented by the general formula (1A) can be widely applied to various reaction methods since the metal complex exhibits excellent reactivity and selectivity as a catalyst for various reactions.

For example, the metal complex represented by the general formula (1A) used in the embodiment of the invention can be used in the oxidation reaction or reduction reaction of an organic compound, and is useful as a catalyst in hydrogenation reduction of aldehydes, ketones, and esters. The metal complex represented by the general formula (1A) used in the embodiment of the invention is useful as a catalyst in dehydrogenation oxidation of amines and alcohols. The metal complex represented by the general formula (1A) used in the embodiment of the invention is also useful as a catalyst in the dehydrogenative oxidation of alcohols, hemiacetals, and hemiaminals, and in an N-alkylation reaction through dehydration condensation of alcohols and amines.

Specific examples of a method to which the catalyst reaction method according to the embodiment of the invention can be applied include reactions such as an oxidation reaction, a reduction reaction, a dehydration condensation reaction, and a hydrolysis reaction.

The catalyst reaction method according to the embodiment of the invention is preferably an oxidation reaction or a reduction reaction of an organic compound.

The oxidation reaction of an organic compound includes, for example, a dehydrogenation reaction.

The reduction reaction of an organic compound includes, for example, a hydrogenation reaction.

The catalyst reaction method according to the embodiment of the invention is preferably a formate forming reaction.

[Catalyst]

In the catalyst reaction method according to the embodiment of the invention, at least one compound selected from the group consisting of a metal complex represented by the following general formula (1A), a tautomer and stereoisomer of the metal complex, and a salt of the metal complex or the tautomer or stereoisomer is used as a catalyst.

[Chem. 7]

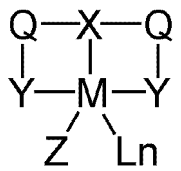

(1A)

(In the general formula (1A), X represents a zero-valent to monovalent atomic group containing a typical element of Groups 13 to 15 and capable of coordinating to M, Q each independently represents a crosslinked structure containing a typical element of Groups 14 to 16 and connecting Y and X, Y each independently represents a zero-valent to monovalent atomic group containing a typical element of Groups 14 to 16 and capable of coordinating to M, M represents a metal atom, Z represents a halogen atom or a hydrogen atom, n represents an integer of 0 to 3, and when more than one L are present, L each independently represents a neutral or anionic ligand.)

The typical element of Groups 13 to 15 of the periodic table in X includes a boron atom, a carbon atom, a silicon atom, a germanium atom, a tin atom, a nitrogen atom, a phosphorus atom, an arsenic atom, an oxygen atom, a sulfur atom, and a selenium atom. The boron atom, the carbon atom, the silicon atom, the germanium atom, the tin atom, the nitrogen atom, the phosphorus atom, and the arsenic atom are preferred. The carbon atom, the nitrogen atom, the phosphorus atom, and the sulfur atom are more preferred. The carbon atom or the nitrogen atom is furthermore preferred.

The atomic group represented by X includes, for example, an alkyl group, an alkenyl group, an alkoxy group, an aromatic ring, and a heterocycle, which may have a substituent or may be combined with other substituents to form a ring.

The alkyl group in X includes a straight chain, branched or cyclic substituted or unsubstituted alkyl group.

The alkyl group in X preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. An alkyl group having 6 or less carbon atoms is preferred, and a methyl group is preferred.

The alkenyl group in X includes a straight chain, branched or cyclic substituted or unsubstituted alkenyl group.

The alkenyl group in X preferably includes an alkenyl group having 2 to 30 carbon atoms, such as a vinyl group, an n-propenyl group, an i-propenyl group, a t-butenyl group, an n-octenyl group. An alkenyl group having 6 or less carbon atoms is preferred.

The alkoxy group in X includes a straight chain, branched or cyclic substituted or unsubstituted alkoxy group.

The alkoxy group in X preferably includes a substituted or unsubstituted alkoxy group having 1 to 30 carbon atoms, such as a methoxy group, an ethoxy group, an isopropoxy group, a t-butoxy group, an n-octyloxy group, or a 2-methoxyethoxy group.

The aromatic ring in X includes, for example, a phenyl ring and a naphthyl ring.

The heterocycle in X includes, for example, a pyrrolidine ring, a piperidine ring, a pyrroline ring, an imidazoline ring, an imidazolidine ring, a pyrrole ring, an imidazole ring, a pyridine ring, a pyrimidine ring, a triazine ring, a quinoline ring, and a quinazoline ring.

The zero-valent to monovalent atomic group represented by X preferably represents an atomic group containing a heteroaromatic ring formed with two carbon atoms and a nitrogen atom, which may have a substituent or may be combined with other substituents to form a ring.

The zero-valent to monovalent atomic group represented by X is preferably a pyrroline ring, a pyridine ring, an imidazoline ring, a pyrimidine ring, or a triazine ring, more preferably a pyridine ring or a triazine ring, and still more preferably a pyridine ring.

When the zero-valent to monovalent atomic group represented by X has a substituent, the substituent includes a substituent group A. An alkyl group is preferred, and a methyl group is more preferred.

The crosslinked structure containing the typical element of Groups 14 to 16 and connecting Y and X represented by Q may have a double bond, may have a monocyclic structure or a condensed ring structure, and may have a substituent.

Although various structures can be introduced into Q in this way, for example, it is preferable that the number of atoms in a part included between Y and X is 1 to 5. 1 to 4 is more preferred, 1 to 3 is furthermore preferred, and 1 to 2 is particularly preferred.

The atom included between the above Y and X is not limited, but a carbon atom, a nitrogen atom, a phosphorus

atom, an oxygen atom, and a sulfur atom are preferred. A carbon atom, a nitrogen atom, and an oxygen atom are more preferred, a carbon atom and a nitrogen atom are furthermore preferable, and a carbon atom is particularly preferable.

Q may have a monocyclic structure. In other words, the crosslinked structure represented by Q may include a ring structure.

When Q has a monocyclic structure, Q may be one in which the monocyclic structure and Y and X in the general formula (1A) are directly bonded, or one in which a divalent substituent may be sandwiched between the monocyclic structure and Y and/or Z in the general formula (1A).

The above divalent substituent includes an alkylene group having 1 to 5 carbon atoms, an alkenylene group having 2 to 5 carbon atoms, a hetero atom such as an oxygen atom or a sulfur atom, a combination of these groups or atoms in series, and the like.

Q each independently preferably represents $CH_2$, NH, or O. $CH_2$ and NH may further have a substituent, and Q is more preferable to represent $CH_2$ or NH.

Q may have a condensed ring structure. In other words, the crosslinked structure represented by Q may include a condensed ring structure.

When Q has a condensed ring structure, Q may be one in which the condensed ring structure and Y and X in the general formula (1A) are directly bonded, or one in which a divalent substituent may be sandwiched between the condensed ring structure and Y and/or X in the general formula (1A).

The above divalent substituent is the same as that described above as the divalent substituent sandwiched between the monocyclic structure and Y and/or X in the general formula (1A).

Q may have a substituent. When Q has neither a monocyclic structure nor a condensed ring structure, the substituent is a substituent for a moiety of Q in the ring structure containing Q, Y, X, and M in the general formula (1A).

When Q has a monocyclic structure or a condensed ring structure, the substituent is a substituent of a monocyclic structure or a condensed ring structure, or another substituent for the moiety of Q in the ring structure including Q, Y, X, and M in the general formula (1A).

The substituent that Q may have, for example, may have a hetero atom, or may be another atom or atomic group.

The substituent having a hetero atom includes an alkoxy group having 1 to 18 carbon atoms, an arylalkoxy group having 7 to 18 carbon atoms, an aryloxy group having 6 to 18 carbon atoms, an acyl group having 2 to 18 carbon atoms, an aroyl group having 7 to 18 carbon atoms, a dialkylamino group having 2 to 18 carbon atoms, an oxygen atom, a sulfur atom, and the like.

The other atoms or atomic groups include, for example, an aromatic group having 3 to 18 carbon atoms, an alkyl group having 1 to 18 carbon atoms, and a halogen atom.

The aromatic group includes, for example, an aryl group having 6 to 20 carbon atoms, such as a phenyl group, an xylyl group, a naphthyl group, and a biphenyl group.

The number of carbon atoms in Q is preferably 12 or less, more preferably 10 or less, and still more preferably 8 or less.

Y each independently represents a zero-valent to monovalent atomic group containing a typical element of Groups 14 to 16 capable of coordinating to M, and may further have a substituent.

The typical element of Groups 14 to 16 is preferably a carbon atom, a nitrogen atom, a phosphorus atom, an arsenic atom, an oxygen atom, a sulfur atom, and a selenium atom, more preferably a carbon atom, a nitrogen atom, a phosphorus atom, and an arsenic atom, still more preferably a nitrogen atom and a phosphorus atom, and particularly preferably a phosphorus atom.

In the general formula (1A), it is preferable that both of Y represent an atomic group containing a nitrogen atom or an atomic group containing a phosphorus atom, or one Y represents an atomic group containing a phosphorus atom and the other Y represents an atomic group containing a nitrogen atom, and it is more preferable that both of Y represent an atomic group containing a phosphorus atom.

When the zero-valent to monovalent atomic group represented by Y has a substituent, the substituent includes the substituent group A. An alkyl group or an aryl group is preferred, and an ethyl group, a t-butyl group, or a phenyl group is more preferred.

M represents a metal atom, and includes, for example, those containing an element from Groups 8 to 11, such as iron, ruthenium, osmium, cobalt, rhodium, iridium, nickel, palladium, platinum, copper, silver, and gold. Among these, iron, ruthenium, cobalt, rhodium, iridium, nickel, palladium, or copper is preferred, ruthenium, rhodium, iridium, nickel, or palladium is more preferred, ruthenium, rhodium, iridium, or palladium is still more preferred, and ruthenium (Ru) is particularly preferred.

Z preferably represents a halogen atom, and more preferably represents a chlorine atom.

n represents an integer of 0 to 3, and represents the number of ligands coordinated to the metal atom represented by M. From the standpoint of catalyst stability, n is preferably an integer of 1 to 3, and more preferably 2 or 3.

When more than one L are present, L each independently represents a neutral or anionic ligand.

The neutral ligand represented by L includes, for example, ammonia, carbon monoxide, phosphines (for

example, triphenylphosphine, or tris(4-methoxyphenyl)phosphine), phosphine oxides (for example, triphenylphosphine oxide), sulfides (for example, dimethyl sulfide), sulfoxides (for example, dimethyl sulfoxide), ethers (for example, diethyl ether), nitriles (for example, p-methylbenzonitrile), and heterocyclic compounds (for example, pyridine, N,N-dimethyl-4-aminopyridine, tetrahydrothiophene or tetrahydrofuran), and is preferably triphenylphosphine.

The anionic ligand represented by L includes, for example, a hydride ion (hydrogen atom), a nitrate ion, and a cyanide ion, and is preferably a hydride ion (hydrogen atom).

In the general formula (1A), X preferably represents a heterocycle, Q preferably represents $CH_2$, NH, or O, Y preferably represents a phosphorus atom, and M preferably represents ruthenium.

Furthermore, Z preferably represents a chlorine atom, n preferably represents 1 to 3, and L each independently preferably represents a hydrogen atom, carbon monoxide, or triphenylphosphine.

In the catalyst reaction method according to the embodiment of the invention, the metal complex represented by the general formula (1A) is preferably a metal complex represented by the following general formula (2A).

[Chem. 8]

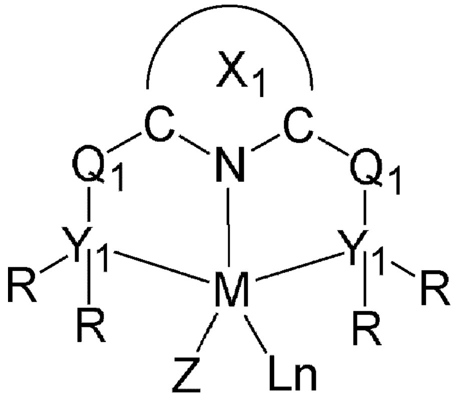

(2A)

(In the general formula (2A), $X_1$ represents a heteroaromatic ring formed with two carbon atoms and a nitrogen atom, which may have a substituent or may be combined with another substituent to form a ring, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

M, Z, n, and L in the general formula (2A) are synonymous with M, Q, Z, n, and L in the general formula (1A), respectively, and preferred ranges thereof are also the same.

The heteroaromatic ring formed with two carbon atoms and a nitrogen atom represented by $X_1$ is preferably a pyrroline ring, a pyridine ring, an imidazoline ring, a pyrimidine ring, or a triazine ring, more preferably a pyridine ring or a triazine ring, and still more preferably a pyridine ring.

The substituent that $X_1$ may have includes the substituent group A. An alkyl group is preferred, and a methyl group is more preferred.

$Q_1$ each independently represents $CH_2$, NH, or O, and more preferably represents $CH_2$ or NH.

The substituents that $CH_2$ and NH may have include the substituents that Q in the general formula (1A) may have.

$Y_1$ each independently represents a phosphorus atom or a nitrogen atom, both of $Y_1$ may represent nitrogen atoms or phosphorus atoms, or one $Y_1$ may represent a phosphorus atom and the other $Y_1$ may represent a nitrogen atom. Both of $Y_1$ are preferably nitrogen atoms or phosphorus atoms, and both of $Y_1$ are more preferably phosphorus atoms.

The alkyl group represented by R includes a straight chain, branched or cyclic substituted or unsubstituted alkyl group. The alkyl group represented by R preferably includes an alkyl group having 1 to 30 carbon atoms, such as a methyl group, an ethyl group, an n-propyl group, an i-propyl group, a t-butyl group, an n-octyl group, an eicosyl group, or a 2-ethylhexyl group. From the standpoint of the catalyst activity, an alkyl group having 12 or less carbon atoms is preferred, an ethyl group or a t-butyl group is preferred, and a t-butyl group is more preferred.

The aryl group represented by R includes a substituted or unsubstituted aryl group having 6 to 30 carbon atoms, such as a phenyl group, a p-tolyl group, a naphthyl group, an m-chlorophenyl group, or an o-hexadecanoylaminophenyl group. An aryl group having 12 or less carbon atoms is preferred, and a phenyl group is more preferred.

The aralkyl group represented by R includes a substituted or unsubstituted aralkyl group having 7 to 30 carbon atoms, such as a trityl group, a benzyl group, a phenethyl group, a tritylmethyl group, a diphenylmethyl group, or a naphthylmethyl group, and is preferably an aralkyl group having 12 or less carbon atoms.

When R further has a substituent, the substituent includes the substituent group A. A methyl group, an ethyl group, an i-propyl group, a t-butyl group, or a phenyl group is preferred, and an ethyl group, an i-propyl group, or a t-butyl group is more preferred.

In the general formula (2A), $X_1$ preferably represents a pyridine ring or a triazine ring, $Q_1$ preferably represents $CH_2$, NH, or O, $Y_1$ preferably represents a phosphorus atom, R preferably represents an ethyl group, a t-butyl group, or a phenyl group, and M preferably represents ruthenium.

Furthermore, Z preferably represents a chlorine atom, n preferably represents an integer of 1 to 3, and L each independently preferably represents a hydrogen atom, carbon monoxide, or triphenylphosphine.

In the catalyst reaction method according to the embodiment of the invention, the metal complex represented by the general formula (2A) is preferably a metal complex represented by the following general formula (3A).

[Chem. 9]

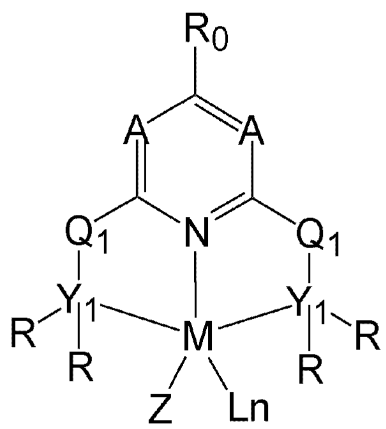

(3A)

(In the general formula (3A), $R_0$ represents a hydrogen atom or an alkyl group, A each independently represents CH, $CR_5$, or N, $R_5$ represents an alkyl group, an aryl group, an aralkyl group, an amino group, a hydroxy group, or an alkoxy group, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

$Y_1$, R, $Q_1$, M, Z, n, and L in the general formula (3A) are synonymous with $Y_1$, R, $Q_1$, M, Z, n, and L in the general formula (2A), respectively, and preferred ranges thereof are also the same.

The $R_0$ in the general formula (3A) represents a hydrogen atom or an alkyl group.

The alkyl group represented by $R_0$ includes a straight chain, branched or cyclic substituted or unsubstituted alkyl group. The alkyl group represented by $R_0$ preferably includes an alkyl group having 1 to 30 carbon atoms, such as a methyl group, an ethyl group, an n-propyl group, an i-propyl group, a t-butyl group, an n-octyl group, an eicosyl group or, a 2-ethylhexyl group. From the standpoint of easy procurement of raw materials, an alkyl group having 6 or less carbon atoms is preferred, and a methyl group is preferred.

The $R_0$ in the general formula (3A) is preferably a hydrogen atom or a methyl group.

A each independently represents CH, $CR_5$, or N, and $R_5$ represents an alkyl group, an aryl group, an aralkyl group, an amino group, a hydroxy group, or an alkoxy group.

The alkyl group represented by $R_5$ includes a straight chain, branched or cyclic substituted or unsubstituted alkyl group. The alkyl group represented by $R_5$ preferably includes an alkyl group having 1 to 30 carbon atoms, such as a methyl group, an ethyl group, an n-propyl group, an i-propyl group, a t-butyl group, an n-octyl group, an eicosyl group, or a 2-ethylhexyl group. From the standpoint of easy procurement of raw materials, an alkyl group having 12 or less carbon atoms is preferred, and a methyl group is preferred.

The aryl group represented by $R_5$ includes a substituted or unsubstituted aryl group having 6 to 30 carbon atoms, such as a phenyl group, a p-tolyl group, a naphthyl group, an m-chlorophenyl group, or an o-hexadecanoylaminophenyl group. An aryl group having 12 or less carbon atoms is preferred, and a phenyl group is more preferred.

The aralkyl group represented by $R_5$ includes a substituted or unsubstituted aralkyl group having 30 or less carbon atoms, such as a trityl group, a benzyl group, a phenethyl group, a tritylmethyl group, a diphenylmethyl group, or a naphthylmethyl group, and is preferably an aralkyl group having 12 or less carbon atoms.

The alkoxy group represented by $R_5$ preferably includes a substituted or unsubstituted alkoxy group having 1 to

30 carbon atoms, such as a methoxy group, an ethoxy group, an isopropoxy group, a t-butoxy group, an n-octyloxy group, or a 2-methoxyethoxy group.

In the general formula (3A), a ring including A preferably represents a pyridine ring or a triazine ring, $Q_1$ preferably represents $CH_2$, NH, or O, $Y_1$ preferably represents a phosphorus atom, R preferably represents an ethyl group, a t-butyl group, or a phenyl group, and M preferably represents ruthenium.

Furthermore, Z preferably represents a chlorine atom, n preferably represents an integer of 1 to 3, and L each independently preferably represents a hydrogen atom, carbon monoxide, or triphenylphosphine.

In the catalyst reaction method according to the embodiment of the invention, the metal complex represented by the general formula (3A) is preferably a ruthenium complex represented by the following general formula (4A).

The ruthenium complex represented by the general formula (4A) is soluble in an organic solvent and insoluble in water, and is suitable as a catalyst in the production of formate. A formate formed by the reaction is easily dissolved in water. Therefore, the separation of the catalyst and the formate is easily achieved by a two-phase system reaction, the catalyst and the formate are respectively easily separated and recovered from the reaction system, and this enables production of a formate in high yield, and an expensive catalyst is easily reused.

[Chem. 10]

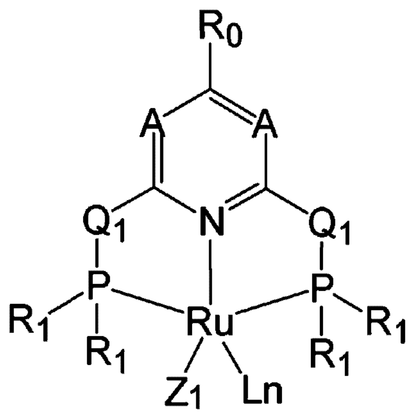

(4A)

(In the general formula (4A), $R_0$ represents a hydrogen atom or an alkyl group, $Q_1$ each independently represents $CH_2$, NH, or O, $CH_2$ and NH may further have a substituent, $R_1$ each independently represents an alkyl group or an aryl group (provided that when $Q_1$ represents NH or O, at least one of $R_1$ represents an aryl group), A each independently represents CH, $CR_5$, or N, $R_5$ represents an alkyl group, an aryl group, an aralkyl group, an amino group, a hydroxy group, or an alkoxy group, $Z_1$ represents a halogen atom, n represents an integer of 0 to 3, and when more than one L are present, L each independently represents a neutral or anionic ligand.)

Ro, A, $Q_1$, L, and N in the general formula (4A) are synonymous with Ro, A, $Q_1$, L, and N in the general formula (3A), respectively, and preferred ranges thereof are also the same.

The alkyl group and aryl group represented by $R_1$ are synonymous with the alkyl group and aryl group represented by R in the general formula (3A), respectively, and preferred ranges thereof are also the same.

$Z_1$ represents a chlorine atom.

The metal complexes represented by the general formulae (1A) to (4A) may form stereoisomers due to coordination of a ligand or conformation, but may be a mixture of these stereoisomers or may be a single pure isomer.

Regarding the metal complexes represented by the general formulae (1A) to (4A), metal complexes produced by a known method or the like can be used. As a known method, for example, a method described in E.Pidko et al., ChemCatChem 2014, 6, 1526 to 1530 or the like can be used.

Specific examples of the metal complexes represented by the general formulae (1A) to (4A) include compounds described below.

In the compounds exemplified below, Et represents an ethyl group, tBu represents a tertiary butyl group, and Ph represents a phenyl group.

[Chem. 11]

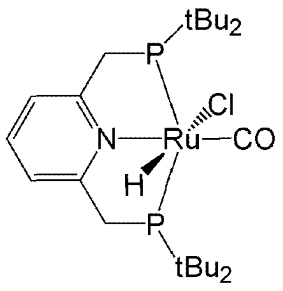

(1)

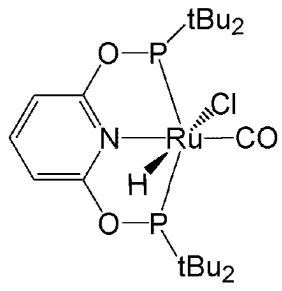

(2)

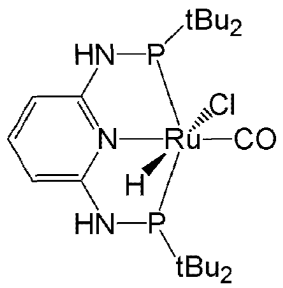

(3)

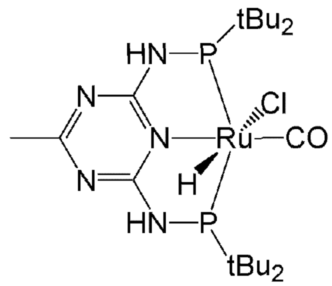

(4)

[Chem. 12]

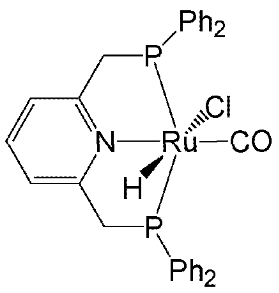

(5)

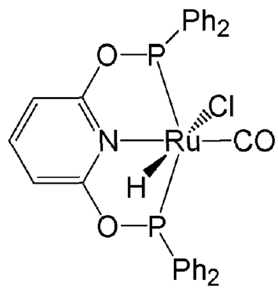

(6)

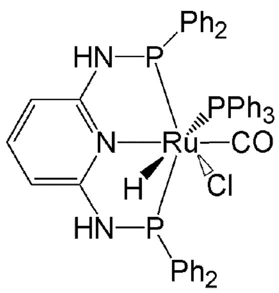

(7)

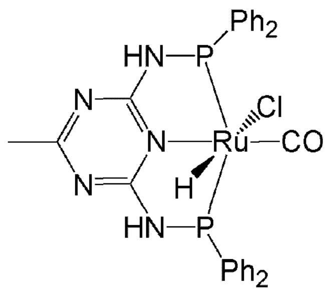

(8)

[Chem. 13]

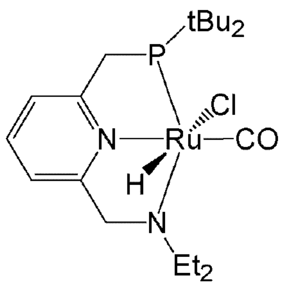

(9)

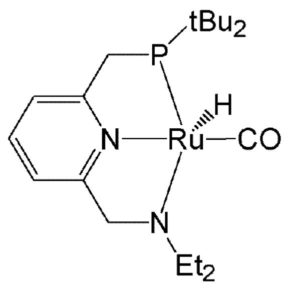

(10)

[Ligand Represented by General Formula (1B)]

In the catalyst reaction method according to the embodiment of the invention, a ligand represented by the following general formula (1B) is used such that a ratio of a substance amount of the ligand to a substance amount of the catalyst in the reaction system is greater than 0 to 15. The ligand represented by the following general formula (1B) may be simply referred to as a ligand hereinafter.

[Chem. 14]

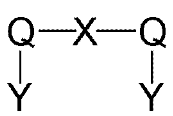

(1B)

(In the general formula (1B), X represents the zero-valent to monovalent atomic group containing the typical

element of Groups 13 to 15 and capable of coordinating to M in the general formula (1A), Q each independently represents a crosslinked structure containing a typical element of Groups 14 to 16 and connecting Y and X, and Y each independently represents the zero-valent to monovalent atomic group containing the typical element of Groups 14 to 16 and capable of coordinating to M in the general formula (1A).)

Y, Q, and X in the general formula (1B) each represent an atomic group or structure corresponding to Y, Q, and X in the general formula (1A), and the preferred ranges also correspond to the same.

In the catalyst reaction method according to the embodiment of the invention, the ligand represented by the general formula (1B) is preferably a ligand represented by the following general formula (2B).

[Chem. 15]

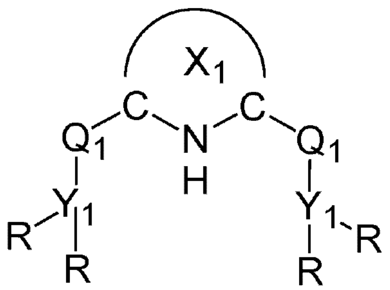

(2B)

(In the general formula (2B), $X_1$ represents a heteroaromatic ring formed with two carbon atoms and a nitrogen atom, which may have a substituent or may be combined with another substituent to form a ring, $Q_1$ each independently represents $CH_2$, NH, or O, $CH_2$ and NH may further have a substituent, $Y_1$ each independently represents a phosphorus atom or a nitrogen atom, and R each independently represents an alkyl group, an aryl group, or an aralkyl group, which may further have a substituent.)

$X_1$, $Q_1$, $Y_1$, and R in the general formula (2B) are synonymous with $X_1$, $Q_1$, $Y_1$, and R in the general formula (2A), respectively, and preferred ranges thereof are also the same.

In the catalyst reaction method according to the embodiment of the invention, the ligand represented by the general formula (2B) is preferably a ligand represented by the following general formula (3B).

[Chem. 16]

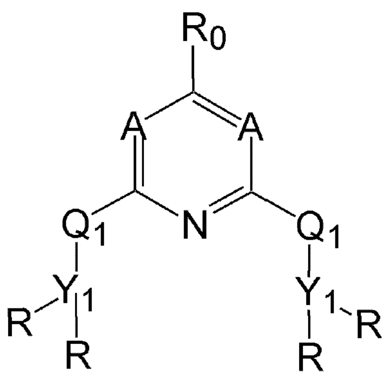

(3B)

(In the general formula (3B), $R_0$ represents a hydrogen atom or an alkyl group, A each independently represents CH, $CR_5$, or N, $R_5$ represents an alkyl group, an aryl group, an aralkyl group, an amino group, a hydroxy group, or an alkoxy group, $Q_1$ each independently represents $CH_2$, NH, or O, $CH_2$ and NH may further have a substituent, $Y_1$ each independently represents a phosphorus atom or a nitrogen atom, and R each independently represents an alkyl group, an aryl group, or an aralkyl group, which may further have a substituent.)

$R_0$, A, $Q_1$, $Y_1$, and R in the general formula (3B) are synonymous with $R_0$, A, $Q_1$, $Y_1$, and R in the general formula (3A), respectively, and preferred ranges thereof are also the same.

In the catalyst reaction method according to the embodiment of the invention, the ligand represented by the general formula (3B) is preferably a ligand represented by the following general formula (4B).

[Chem. 17]

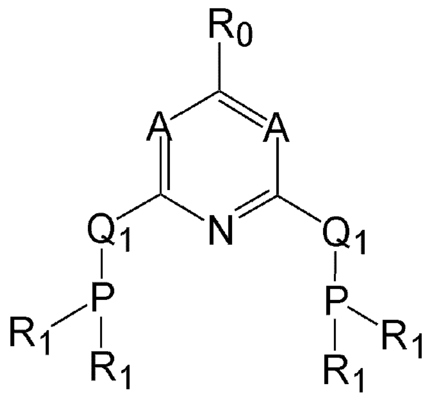

(4B)

(In the general formula (4B), $R_0$ represents a hydrogen atom or an alkyl group, $Q_1$ each independently represents $CH_2$, NH, or O, $CH_2$ and NH may further have a substituent, $R_1$ each independently represents an alkyl group or an aryl group (provided that when $Q_1$ represents NH or O, at least one of $R_1$ represents an aryl group), A each independently represents CH, $CR_5$, or N, and $R_5$ represents an alkyl group, an aryl group, an aralkyl group, an amino group, a hydroxyl group, or an alkoxy group.)

Ro, A, $Q_1$, and $R_1$ in the general formula (4B) are synonymous with Ro, A, $Q_1$, and $R_1$ in the general formula (4A), respectively, and preferred ranges thereof are also the same.

The alkyl group and aryl group represented by $R_1$ are synonymous with the alkyl group and aryl group represented by R in the general formula (3A), respectively, and preferred ranges thereof are also the same.

Specific examples of the ligands represented by the general formulae (1B) to (4B) include compounds described below.

In the compounds exemplified below, Et represents an ethyl group, tBu represents a tertiary butyl group, and Ph represents a phenyl group.

[Chem. 18]

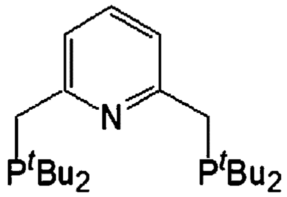

(A)

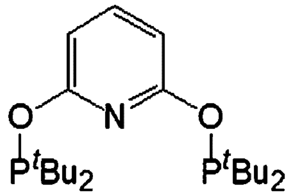

(B)

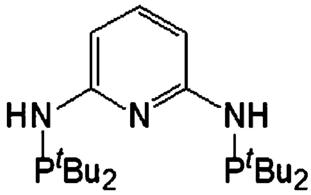

(C)

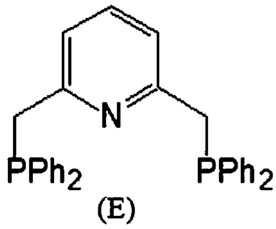

(E)

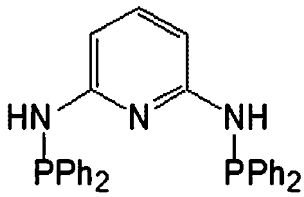

(G)

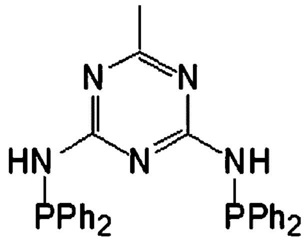

(H)

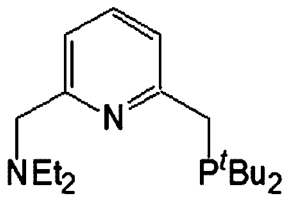

(I)

Regarding the ligands represented by the general formulae (1B) to (4B), ligands produced by a known method or the like can be used. As a known method, for example, a method described in E.Pidko et al., ChemCatChem 2014, 6, 1526 to 1530 or the like can be used.

(Solvent)

The catalyst reaction method according to the embodiment of the invention may be carried out without a solvent or with a solvent. When a solvent is used, the type thereof is not particularly limited as long as the solvent is an inert solvent that does not directly participate in the reaction, and examples thereof include water, methanol, ethanol, ethylene glycol, glycerin, toluene, benzene, xylene, propylene carbonate, dioxane, dimethyl sulfoxide, and methylcyclohexane.

In the catalyst reaction method according to the embodiment of the invention, it is preferable to use a solvent that can be obtained as a two-phase system in which an organic solvent and an aqueous solvent are present in a separated state. It is preferable to include a solvent that dissolves the catalyst to become uniform, and it is more preferable to include a solvent that dissolves the catalyst and the ligand to become uniform.

The aqueous solvent includes, for example, water, methanol, ethanol, ethylene glycol, glycerin, and mixed solvents thereof. Water is preferred from the standpoint of low environmental load.

The organic solvent includes, for example, toluene, benzene, xylene, propylene carbonate, dioxane, dimethyl sulfoxide, and mixed solvents thereof. It is preferable to include toluene or dioxane from the standpoint of separability from the aqueous solvent, and toluene is more preferred.

(Reaction Conditions)

Reaction conditions in the catalyst reaction method according to the embodiment of the invention are not particularly limited, and can be appropriately selected depending on the type of the reaction. The reaction conditions can be appropriately changed during the reaction process. The form of the reaction vessel used for the reaction is not particularly limited.

A method of introducing the catalyst and ligand into the reaction vessel and the order of introduction are not particularly limited.

For example, the catalyst and the ligand may be introduced simultaneously. Furthermore, introduction of the catalyst and the ligand is such that introduction of one or both of the catalyst and the ligand may be conducted continuously or intermittently.

For example, the ligands represented by the general formulae (1B) to (4B) may be added in entirety to the reaction mixture at the start of the reaction, may be added several times, or may be added such that the ratio of the substance amount of the ligand to the substance amount of the catalyst in the reaction system is in the range of greater than 0 to 15.

There is no particular limit to the combination of the metal complex and the ligand used in the reaction, and one type or more types of each may be used, as long as the ratio of the substance amount of the total amount of the ligand to the total amount of the catalyst is in the range of greater than 0 to 15.

A reaction temperature in the catalyst reaction method is not particularly limited, and is preferably 30°C or higher, more preferably 40°C or higher, and still more preferably 50°C or higher in order to efficiently progress the reaction. Furthermore, from the standpoint of energy efficiency, the reaction temperature is preferably 200°C or lower, more preferably 150°C or lower, and still more preferably 100°C or lower

The reaction temperature can be adjusted by heating or cooling, and temperature increase by heating is preferred.

For example, in the reaction of hydrogen with carbon dioxide, for example, the temperature may be increased by heating after introducing hydrogen and carbon dioxide into a reaction vessel, or hydrogen may be introduced after introducing carbon dioxide into a reaction vessel and increasing the temperature.

A reaction time in the catalyst reaction method is not particularly limited, and is, for example, preferably 0.5 hours or longer, more preferably 1 hour or longer, and still more preferably 2 hours or longer. Furthermore, the reaction time is preferably 24 hours or less, more preferably 12 hours or less, and still more preferably 6 hours or less, from the standpoint of cost.

[Formate Forming Reaction, Method for Producing Formate, and Method for Producing Formic Acid]

As described above, the catalyst reaction method according to the embodiment of the invention may be a formate forming reaction.

Hereinafter, a case where the catalyst reaction method according to the embodiment of the invention is a formate forming reaction will be described in detail.

When the catalyst reaction method according to the embodiment of the invention is applied to the formate forming reaction, there is an advantage that a stable formate can be produced.

In the embodiment of the invention, the formate forming reaction is preferably a method of producing a formate by allowing hydrogen to react with at least one compound selected from the group consisting of carbon dioxide, a hydrogen carbonate, and a carbonate in the presence of a solvent by using a catalyst.

The reaction in the formate forming reaction is preferably a two-phase system in which an organic solvent and an aqueous solvent are present in a separated state in the solvent.

That is, the above formate forming reaction is a method for producing a formate by reacting hydrogen with at least one compound selected from the group consisting of carbon dioxide, a hydrogen carbonate, and a carbonate in the presence of a solvent, and

the above formate forming reaction is a two-phase system reaction in which an organic solvent and an aqueous solvent are present in a separated state in the solvent.

The method for producing a formate according to the embodiment of the invention includes a first step of producing the formate by the above catalyst reaction method.

The above formate forming reaction may be the first step.

A method for producing formic acid according to the embodiment of the invention includes:

a first step of producing a formate by the above catalyst reaction method, and
a second step of protonating at least a part of the formate to form formic acid.

The second step according to the embodiment of the invention is a step of protonating at least a part of the formate by electrodialysis to form formic acid and water.

<First Step>

The first step is a step of producing a formate by the above catalyst reaction method.
That is, the first step is a step of producing a formate by the catalyst reaction method using a ligand represented by the above general formula (1B) such that the ratio of the substance amount of the ligand to the substance amount of the catalyst in the reaction system is greater than 0 to 15 in the catalyst reaction using the metal complex represented by the above general formula (1A) as the catalyst.
The first step may be a step of allowing hydrogen to react with at least one compound selected from the group consisting of carbon dioxide, a bicarbonate, a hydrogen carbonate, and a carbonate in the presence of a solvent using the ligand represented by the general formula (1B) such that the ratio of the substance amount of the ligand to the substance amount of the catalyst in the reaction system is greater than 0 to 15, thereby producing a formate in the reaction solution.
In the embodiment of the invention, the reaction of hydrogen with at least one compound selected from the group consisting of carbon dioxide, a hydrogen carbonate, and a carbonate is preferably carried out in a two-phase system in which an organic solvent and an aqueous solvent are present in a separated state in the solvent, and is preferably carried out in a solution containing an organic solvent in which the catalyst is dissolved.
The method for producing a formate according to the embodiment of the invention can be carried out, for example, as follows. A reaction vessel equipped with a stirring device is provided, and a solvent is introduced into the reaction vessel. As necessary, a phase transfer catalyst may be further added. A catalyst and a ligand are added to the reaction vessel and dissolved in a solvent to prepare a catalyst solution. Hydrogen and at least one compound selected from the group consisting of carbon dioxide, a bicarbonate, a hydrogen carbonate, and a carbonate are introduced into the reaction vessel, and the reaction is carried out. As necessary, a ligand may be further added.

(Solvent)

The solvent used in the embodiment of the invention is preferably one that can be obtained as a two-phase system in which an organic solvent and an aqueous solvent are present in a separated state, preferably includes a solvent that dissolves the catalyst to form a uniform solution, and more preferably includes a solvent that can dissolve the catalyst and the ligand to form a homogeneous system.
The aqueous solvent includes, for example, water, methanol, ethanol, ethylene glycol, glycerin, and mixed solvents thereof. Water is preferred from the standpoint of low environmental load.
The organic solvent includes, for example, toluene, benzene, xylene, propylene carbonate, dioxane, dimethyl sulfoxide, and mixed solvents thereof. It is preferable to include toluene or dioxane from the standpoint of separability from the aqueous solvent, and toluene is more preferred.

(Catalyst)

In the method for producing a formate according to the embodiment of the invention as described above, in a catalyst reaction using, as a catalyst, at least one compound selected from the group consisting of a metal complex represented by the following general formula (1A), a tautomer or stereoisomer of the metal complex, and a salt of the metal complex or the tautomer or stereoisomer, a ligand represented by the above general formula (1B) is used in such a manner that a ratio of a substance amount of the ligand to a substance amount of the catalyst in the reaction system is greater than 0 to 15.
In the method for producing a formate according to the embodiment of the present invention, the metal complex represented by the general formula (1A) and the ligand represented by the general formula (1B) are the same as those described above in the catalyst reaction method according to the embodiment of the present invention, and the preferred ones are also the same.
The metal complex represented by the general formula (1A) is dissolved in an organic solvent and is insoluble in water. A formate formed by the reaction is easily dissolved in water. Therefore, the separation of the catalyst and the formate is easily achieved by a two-phase system reaction, the catalyst and the formate are respectively easily separated and recovered from the reaction system, and this enables production of a formate in high yield.
According to the method of the present embodiment, a formate formed by the reaction can be separated from the catalyst by simple operation, and an expensive catalyst is easily reused.

(Phase Transfer Catalyst)

The catalyst reaction method according to the embodiment and the method for producing a formate according to the embodiment of the invention preferably conduct the reaction in a two-phase system, and may use a phase transfer catalyst in order to smoothly perform the transfer of a substance between two phases. The phase transfer catalyst includes, for example, a quaternary ammonium salt (ammonium salt), a quaternary phosphate, a macrocyclic polyether such as a crown ether, a nitrogen-containing macrocyclic polyether such as a cryptand, a nitrogen-containing chain polyether, polyethylene glycol, and an alkyl ether thereof. Above all, a quaternary ammonium salt is preferred from the standpoint that mass transfer between an aqueous solvent and an organic solvent is easy even under mild reaction conditions.
The quaternary ammonium salt includes, for example, methyltrioctylammonium chloride, benzyltrimethylammonium chloride, benzyltriethylammonium chloride, tetrabutylammonium hydroxide, tetrabutylammonium fluoride, tetrabutylammonium bromide, tetrabutylammonium iodide, trimethylphenylammonium bromide, tributylammonium tribromide, tetrahexylammonium hydrogen sulfate, decyltrimethylammonium bromide, diallyldimethylammonium chloride, dodecyltrimethylammonium bromide, dimethyldioctadecylammonium bromide, tetraethylammonium tetrafluoroborate, ethyltrimethylammonium iodide tris(2-hydroxyethyl)methylammonium hydroxide, tetramethylammonium acetate, tetramethylammonium bromide, and tetraethylammonium iodide. Methyltrioctylammonium chloride is preferred.
The amount of the phase transfer catalyst used is not particularly limited so long as a formate can be produced. The amount of the phase transfer catalyst used is preferably 0.1 mmol or more, more preferably 0.5 mmol or more, and still more preferably 1 mmol or more, per 1 L of the organic phase and aqueous phase solvents for the purpose of efficiently supporting the transfer of a reaction substrate such as a carbonate or a hydrogen carbonate. Furthermore, from the standpoint of cost, the amount is preferably 1 mol or less, more preferably 500 mmol or less, and still more preferably 100 mmol or less. When two or more kinds of the phase transfer catalysts are used, the total amount of those needs to be in the above range.

(Carbon Dioxide and Hydrogen)

As hydrogen used in the embodiment of the invention, either a hydrogen gas cylinder or liquid hydrogen can be used. As a hydrogen supply source, for example, hydrogen generated during a smelting process of iron manufacture, hydrogen generated during a soda manufacturing process, and the like can be used. Furthermore, hydrogen generated from electrolysis of water can be used.
Carbon dioxide used in the embodiment of the invention may be pure carbon dioxide gas or may be a mixed gas containing a component other than carbon dioxide. Carbon dioxide gas and other gas may be separately introduced, and a mixed gas may be formed beforehand and introduced.
The component other than carbon dioxide includes an inert gas such as nitrogen or argon, water vapor, and any optional component contained in an exhaust gas or the like.
As the carbon dioxide, a carbon dioxide gas cylinder, liquid carbon dioxide, supercritical carbon dioxide, dry ice, and the like can be used.
Hydrogen gas and carbon dioxide gas may be introduced into the reaction system each alone or may be introduced as a mixed gas.
The proportions of hydrogen and carbon dioxide used are preferably such that the proportions are equal amounts on a molar basis or hydrogen is in excess.
When a hydrogen cylinder is used as the hydrogen used in the method for producing a formate according to the embodiment of the invention, the pressure is preferably 0.1 MPa or more, more preferably 0.2 MPa or more, and still more preferably 0.5 MPa or more, from the standpoint of sufficiently securing reactivity. Furthermore, the pressure is preferably 50 MPa or less, more preferably 20 MPa or less, and still more preferably 10 MPa or less, from the standpoint that facilities are liable to become large.
The pressure of carbon dioxide used in the method for producing a formate according to the embodiment of the invention is preferably 0.1 MPa or more, more preferably 0.2 MPa or more, and still more preferably 0.5 MPa or more, from the standpoint of sufficiently securing reactivity. Furthermore, the pressure is preferably 50 MPa or less, more preferably 20 MPa or less, and still more preferably 10 MPa or less, from the standpoint that facilities are liable to become large.
Hydrogen gas and carbon dioxide gas may be introduced into a catalyst solution by bubbling (blowing). Furthermore, after introducing a gas including hydrogen gas and carbon dioxide gas, the catalyst solution, hydrogen gas, and carbon dioxide gas may be stirred by stirring with a stirring device or by rotating the reaction vessel.
A method for introducing carbon dioxide, hydrogen, a catalyst, a solvent, and the like that are used for the reaction into the reaction vessel is not particularly limited. All of the raw materials may be introduced at once, a part or all of the raw materials may be introduced stepwise, or a part or all of the raw materials may be introduced continuously. Furthermore, the method may be an introduction method combining those methods.

(Hydrogen Carbonate and Carbonate)

The hydrogen carbonate and carbonate used in the embodiment of the invention include a carbonate or hydrogen carbonate of an alkali metal or an alkaline earth metal.

The hydrogen carbonate includes, for example, sodium hydrogen carbonate and potassium hydrogen carbonate. Potassium hydrogen carbonate is preferred from the standpoint of high solubility in water.

The carbonate includes, for example, sodium carbonate, potassium carbonate, sodium potassium carbonate, and sodium sesquicarbonate.

The hydrogen carbonate and carbonate can be formed by a reaction of carbon dioxide with a base. For example, the hydrogen carbonate or carbonate may be formed by introducing carbon dioxide into a basic solution.

The solvent of the basic solution for forming the hydrogen carbonate or carbonate is not particularly limited, and the solvent includes water, methanol, ethanol, N,N-dimethylformamide, dimethyl sulfoxide, tetrahydrofuran, benzene, toluene, mixed solvents of these, and the like. The solvent preferably includes water and is more preferably water.

The base used for the basic solution is not particularly limited so long as the base can react with carbon dioxide and form a hydrogen carbonate or carbonate. The base is preferably a hydroxide. For example, the base includes at least one selected from lithium hydrogen carbonate, sodium hydrogen carbonate, potassium hydrogen carbonate, cesium hydrogen carbonate, potassium hydroxide, sodium hydroxide, diazabicycloundecene, and triethylamine. Among those described above, the base is preferably a hydroxide, more preferably potassium hydroxide or sodium hydroxide, and still more preferably potassium hydroxide.

The content of the base in the basic solution is not particularly limited so long as a hydrogen carbonate and a carbonate can be produced. The content of the base is preferably 0.1 mol or more, more preferably 0.5 mol or more, and still more preferably 1 mol or more, per 1 L of the aqueous phase solvent, from the standpoint of securing the amount of the formate produced. Furthermore, the content is preferably 30 mol or less, more preferably 20 mol or less, and still more preferably 15 mol or less, from the standpoint of the reaction efficiency for the formate. However, when the content exceeds the solubility in the aqueous phase, the solution becomes turbid.

The ratio of the amounts of carbon dioxide and a base used for the reaction of carbon dioxide and a base is preferably 0.1 or more, more preferably 0.5 or more, and still more preferably 1.0 or more, as a molar ratio, from the standpoint of forming a carbonate from carbon dioxide. Furthermore, the ratio is preferably 8.0 or less, more preferably 5.0 or less, and still more preferably 3.0 or less, from the standpoint of the efficiency of utilization of carbon dioxide.

The ratio of the amounts of carbon dioxide and a base used may be a ratio of molar amounts of carbon dioxide and the base that are introduced into the reaction vessel, and is molar amount (mol) of $CO_2$ / molar amount (mol) of the base.

When the ratio of the amounts of carbon dioxide and the base used is in the above range, excessive input of carbon dioxide into the reaction vessel is suppressed, unreacted carbon dioxide can be suppressed to a minimal level, and the final efficiency of formic acid conversion is easily improved. Furthermore, from the reaction between carbon dioxide and the base in the same vessel, carbon dioxide is hydrogenated via a hydrogen carbonate or a carbonate, and a formate can be formed.

Unreacted carbon dioxide can be recovered from the reaction vessel and reused.

The method of introducing carbon dioxide and a base into a reaction vessel and the order of introduction are not particularly limited. However, carbon dioxide is preferably introduced after the base is introduced into the reaction vessel. Furthermore, the introduction of carbon dioxide and the base is such that introduction of one or both of carbon dioxide and the base may be conducted continuously or intermittently.

The reaction temperature in the reaction for forming a hydrogen carbonate or a carbonate by a reaction between carbon dioxide and a base is not particularly limited. However, the reaction temperature is preferably 0°C or higher, more preferably 10°C or higher, and still more preferably 20°C or higher, in order to dissolve carbon dioxide in the aqueous phase. Furthermore, the reaction temperature is preferably 100°C or lower, more preferably 80°C or lower, and still more preferably 40°C or lower

The reaction time in the reaction for forming a hydrogen carbonate or a carbonate by a reaction between carbon dioxide and a base is not particularly limited. However, for example, the reaction time is preferably 0.5 hours or more, more preferably 1 hour or more, and still more preferably 2 hours or more, from the standpoint of sufficiently securing the amount of the hydrogen carbonate or carbonate formed. Furthermore, the reaction time is preferably 24 hours or less, more preferably 12 hours or less, and still more preferably 6 hours or less, from the standpoint of cost.

The hydrogen carbonate and carbonate formed by the reaction of carbon dioxide and a base can be used for a reaction of hydrogen with a hydrogen carbonate or a carbonate in the method for producing a formate according to the embodiment of the invention. Furthermore, formation of a hydrogen carbonate or a carbonate by the reaction of carbon dioxide with a base in the reaction vessel may be conducted as introduction of a hydrogen carbonate or a carbonate in the reaction vessel in the method for producing a formate.

(Reaction Conditions)

The reaction conditions in the method for producing a formate according to the embodiment of the invention are not particularly limited, and the reaction conditions can be appropriately changed during the reaction process. The form of the reaction vessel used for the reaction is not particularly limited.

The reaction of hydrogen with at least one compound selected from the group consisting of carbon dioxide, a hydrogen carbonate, and a carbonate in the method for producing a formate according to the embodiment of the invention includes a reaction of hydrogen with carbon dioxide, a reaction of hydrogen with a hydrogen carbonate, and a reaction of hydrogen with a carbonate.

In the reaction of hydrogen with carbon dioxide, a reaction of carbonation of carbon dioxide and a reaction for forming a formate by hydrogenation of a carbonate proceed simultaneously.

The method of introducing hydrogen, carbon dioxide, a hydrogen carbonate, or a carbonate into a reaction vessel and the order of introduction are not particularly limited.

For example, in the reaction of hydrogen and carbon dioxide, hydrogen and carbon dioxide are preferably introduced simultaneously. Hydrogen and carbon dioxide may be introduced singly or may be introduced as a mixed gas. Furthermore, introduction of hydrogen and carbon dioxide is such that introduction of one or both of hydrogen and carbon dioxide may be conducted continuously or intermittently.

In the reaction of hydrogen with a hydrogen carbonate and the reaction of hydrogen with a carbonate, hydrogen is preferably introduced after the hydrogen carbonate or carbonate is introduced into the reaction vessel. Introduction of hydrogen and a hydrogen carbonate or a carbonate is such that introduction of one or both of hydrogen and a hydrogen carbonate or a carbonate may be conducted continuously or intermittently.

The reaction temperature in the reaction of hydrogen with at least one compound selected from the group consisting of carbon dioxide, a hydrogen carbonate, and a carbonate is not particularly limited. However, in order to efficiently proceed the reaction, the reaction temperature is preferably 30°C or higher, more preferably 40°C or higher, and still more preferably 50°C or higher Furthermore, from the standpoint of energy efficiency, the reaction temperature is preferably 200°C or lower, more preferably 150°C or lower, and still more preferably 100°C or lower.

The reaction temperature can be adjusted by heating or cooling, and temperature increase by heating is preferred.

Furthermore, in the reaction of hydrogen with carbon dioxide, for example, the temperature may be increased by heating after introducing hydrogen and carbon dioxide into a reaction vessel, or hydrogen may be introduced after introducing carbon dioxide into a reaction vessel and increasing the temperature.

In the reaction of hydrogen with a hydrogen carbonate or a carbonate, for example, hydrogen may be introduced after introducing (forming) the hydrogen carbonate or carbonate into (in) the reaction vessel, and the temperature may be increased. The hydrogen may be introduced after introducing (forming) the hydrogen carbonate or carbonate into (in) the reaction vessel and increasing the temperature

The reaction time in the reaction of hydrogen with at least one compound selected from the group consisting of carbon dioxide, a hydrogen carbonate, and a carbonate is not particularly limited. However, for example, the reaction time is preferably 0.5 hours or more, more preferably 1 hour or more, and still more preferably 2 hours or more, from the standpoint of sufficiently securing the amount of a formate formed. Furthermore, the reaction time is preferably 24 hours or less, more preferably 12 hours or less, and still more preferably 6 hours or less, from the standpoint of cost.

<Second Step>

The first step may be followed by the second step of protonating at least a part of the formate to form formic acid and water. Protonation is preferably carried out by electrodialysis.

In the embodiment of the invention, since the formate formed in the first step is eluted into the aqueous phase, an aqueous solution of formate is obtained by fractionating the aqueous phase.

Formic acid can be formed by separating the aqueous phase in the first step and treating the obtained aqueous solution of formate using an electrodialyzer by the second step. The aqueous phase to be separated is preferably the aqueous phase after completion of the first step.

In the second step, the aqueous solution of formate obtained by the first step as described above may be used as it is, or the formate concentration may be adjusted by concentrating or diluting the solution as necessary and used.

A method for diluting an aqueous solution of a formate includes a method of diluting the solution by adding pure water.

A method for concentrating an aqueous solution of a formate includes a method of distilling off water from the aqueous solution of formate, a method of concentrating the aqueous solution of formate using a separation membrane unit equipped with a reverse osmosis membrane, and the like.

During the treatment using an electrodialyzer, it is preferable to separate the aqueous phase in the first step,

adjust the concentration of the formate in the aqueous phase by dilution and then use the aqueous phase in the second step, from the standpoint of suppressing the formate loss caused by a phenomenon of concentration diffusion of a high concentration aqueous solution of a formate.

When an aqueous solution of a formate with high concentration is obtained by the first step, the formate concentration is adjusted by diluting to a concentration appropriate for electrodialysis, and then the aqueous solution of formate is supplied to the second step, formic acid can be produced in higher yield and with more excellent productivity.

The degree of adjustment (preferably dilution) of the concentration of the aqueous solution of formate obtained in the first step can be appropriately selected. The concentration of a formate in the aqueous solution of formate after concentration adjustment is preferably a concentration appropriate for electrodialysis, preferably 2.5 mol/L or more, more preferably 3 mol/L or more, more preferably 4.75 mol/L or more, and still more preferably 5 mol/L or more. Furthermore, the concentration is preferably 20 mol/L or less, more preferably 15 mol/L or less, and still more preferably 10 mol/L or less, from the standpoint of preventing the formate loss caused by a phenomenon of concentration diffusion of the high-concentration aqueous solution of formate during a treatment using an electrodialyzer.

Pure water can be used for dilution. Furthermore, the water formed in the second step may be used for dilution. It is preferable to reuse the water formed by the second step during dilution because there are advantages such that the cost required for waste water treatment and the environmental load can be reduced.

In the method for producing formic acid according to the embodiment of the invention, the aqueous solution of formate obtained by the first step may be reused in the second step after adding acid to the solution and conducting a decarbonation treatment. That is, the aqueous phase in the first step is separated, acid is added to conduct a decarbonation treatment, and then the aqueous phase may be used in the second step.

The aqueous solution of formate obtained by the first step may include unreacted carbonate and hydrogen carbonate formed by side reactions, and when the solution containing carbonate and hydrogen carbonate is electrodialyzed, there is a risk that carbon dioxide may be generated, and the dialysis efficiency may be decreased. Therefore, by adding acid to the aqueous solution of formate obtained by the first step, conducting a decarbonation treatment and then electrodialyzing the solution, TON (Turnover Number) can be further increased, and formic acid can be produced in higher yield with more excellent productivity.

The acid used for the decarbonation treatment includes, for example, formic acid, citric acid, acetic acid, malic acid, lactic acid, succinic acid, tartaric acid, butyric acid, fumaric acid, propionic acid, hydrochloric acid, nitric acid, and sulfuric acid. Formic acid is preferably used.

Regarding the amount of the acid used, the amount of the acid used is preferably 50% or more, and more preferably 80% or more, with respect to the amount of carbonic acid present in the solution, from the standpoint of suppressing the amount of carbonic acid generated during the electrodialysis treatment. Furthermore, from the standpoint of suppressing deterioration of the electrodialyzer by making the pH of the formate solution neutral during the electrodialysis treatment, the amount of the acid used is preferably 150% or less, and more preferably 120% or less, with respect to the amount of carbonic acid present in the solution.

In the embodiment of the invention, the proportion of the formate that is protonated by the second step is such that the proportion to be protonated is preferably 10% or more, the proportion to be protonated is more preferably 20% or more, and the proportion to be protonated is still more preferably 30% or more, with respect to the initial molar amount of the formate in the aqueous solution of formate, from the standpoint of increasing the purity of the aqueous solution of formic acid recovered.

The electrodialyzer includes a two-chamber type electrodialyzer that uses a bipolar membrane and an anionic exchange membrane or a cationic exchange membrane, a three-chamber type electrodialyzer that uses a bipolar membrane, an anionic exchange membrane and a cationic exchange membrane, and the like.

The three-chamber electrodialyzer is equipped with more than one bipolar membrane, more than one anionic exchange membrane, and more than one cationic exchange membrane, respectively, and these bipolar membranes, anionic exchange membranes, and cationic exchange membranes are disposed between an anode and a cathode, forming a base tank, a sample tank (salt tank), and an acid tank. As an aqueous solution of a formate is circulated and supplied to the sample tank while passing electricity, the formate is gradually converted to formic acid, formic acid is recovered from the acid tank, water is recovered from the sample tank, and hydroxide is recovered from the base tank.

A two-chamber type electrodialyzer is equipped with more than one bipolar membrane and more than one cationic exchange membrane, respectively, and these bipolar membranes and cationic exchange membranes are alternately disposed between an anode and a cathode. Each salt chamber is formed between each bipolar membrane and a cationic exchange membrane disposed on the cathode side thereof, while a base tank is formed between each bipolar membrane and a cationic exchange membrane disposed on the anode side thereof. By circulating and supplying an aqueous solution of a formate to the salt chamber while passing electricity, the formate circulated and supplied to the salt chamber is gradually converted to formic acid while forming hydroxide in the base tank.

A formic acid solution can be obtained by protonating the formate by a simple method by the second step.

In the embodiment of the invention, the metal complex represented by the general formula (1A) can be subjected

to a post-treatment, isolation, and refinement, as necessary. Specific examples of the post-treatment method include concentration, solvent substitution, cleaning, extraction, back extraction, filtration, and crystallization by addition of poor solvent. These can be conducted singly or in combination. Specific examples of the isolation and refinement methods include drying of a reaction solution, column chromatography, recrystallization, and crystal cleaning with poor solvent. These can be conducted singly or in combination.

The metal complex represented by the general formula (1A) is suitable for industrial use, and the reaction can be conducted under mild reaction conditions with high catalyst activity. For example, a formate can be produced by hydrogenation reduction of carbon dioxide or a hydrogen carbonate in the presence of a hydrogen donor.

As described above, the following matters are disclosed in the present specification.

[1] A catalyst reaction method, in which
in a catalyst reaction using at least one catalyst selected from the group consisting of a metal complex represented by the following general formula (1A), a tautomer or stereoisomer of the metal complex, and a salt of the metal complex or the tautomer or stereoisomer, a ligand represented by the following general formula (1B) is used in such a manner that a ratio of a substance amount of the ligand to a substance amount of the catalyst in a reaction system is greater than 0 to 15.

[Chem. 19]

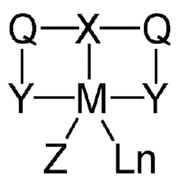

(1A)

(In the general formula (1A), X represents a zero-valent to monovalent atomic group containing a typical element of Groups 13 to 15 and capable of coordinating to M, Q each independently represents a crosslinked structure containing a typical element of Groups 14 to 16 and connecting Y and X, Y each independently represents a zero-valent to monovalent atomic group containing a typical element of Groups 14 to 16 and capable of coordinating to M, M represents a metal atom, Z represents a halogen atom or a hydrogen atom, n represents an integer of 0 to 3, and when more than one L are present, L each independently represents a neutral or anionic ligand.)

[Chem. 20]

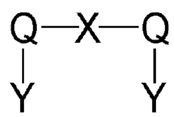

(1B)

(In the general formula (1B), X represents the zero-valent to monovalent atomic group containing the typical element of Groups 13 to 15 and capable of coordinating to M in the general formula (1A), Q each independently represents a crosslinked structure containing a typical element of Groups 14 to 16 and connecting Y and X, and Y each independently represents the zero-valent to monovalent atomic group containing the typical element of Groups 14 to 16 and capable of coordinating to M in the general formula (1A).)

[2] The catalyst reaction method described in [1], in which
the metal complex represented by the general formula (1A) is a metal complex represented by the following general formula (2A).

[Chem. 21]

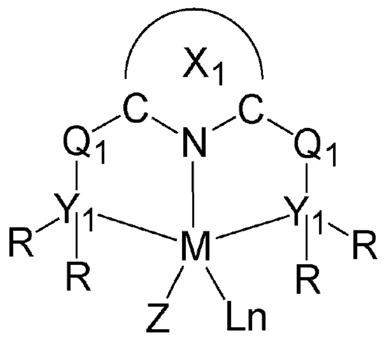

(2A)

(In the general formula (2A), $X_1$ represents a heteroaromatic ring formed with two carbon atoms and a nitrogen atom, which may have a substituent or may be combined with another substituent to form a ring, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

[3] The catalyst reaction method described in [2], in which

the metal complex represented by the general formula (2A) is a metal complex represented by the following general formula (3A).

[Chem. 22]

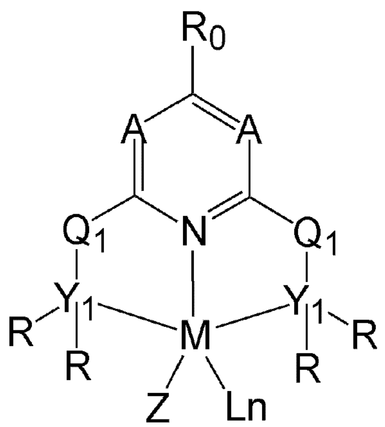

(3A)

(In the general formula (3A), $R_0$ represents a hydrogen atom or an alkyl group, A each independently represents CH, $CR_5$, or N, $R_5$ represents an alkyl group, an aryl group, an aralkyl group, an amino group, a hydroxy group, or an alkoxy group, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand.)

[4] The catalyst reaction method described in any one of [1] to [3], in which the metal atom represented by M is ruthenium.

[5] The catalyst reaction method described in any one of [1] to [4], in which the catalyst reaction is an oxidation reaction or a reduction reaction of an organic compound.

[6] The catalyst reaction method described in any one of [1] to [4], in which the catalyst reaction is a formate forming reaction.

[7] The catalyst reaction method described in [6], in which

the formate forming reaction is a method for producing a formate by allowing hydrogen to react with at least one

compound selected from the group consisting of carbon dioxide, a hydrogen carbonate, and a carbonate in the presence of a solvent, and
the formate forming reaction is a two-phase system reaction in which an organic solvent and an aqueous solvent are present in a separated state in the solvent.

[8] A method for producing a formate, the method including:
a first step of producing the formate by the catalyst reaction method described in [6] or [7].
[9] A method for producing formic acid, the method including:

a first step of producing a formate by the catalyst reaction method described in [6] or [7]; and
a second step of protonating at least a part of the formate to form formic acid.

EXAMPLES

The invention is described in detail below by reference to Examples and Comparative Examples. However, it should be understood that the invention is not limited to those Examples.

[Synthesis of Catalyst]

(Synthesis Example 1) Synthesis of Ru Catalyst 1

Ru catalyst 1 was synthesized by the following operation.
40 mg (0.1 mmol) of a ligand A shown below was added to a THF (tetrahydrofuran) (5 ml) suspension of 95.3 mg (0.1 mmol) of $[RuHCl(PPh_3)_3(CO)]$ in an inert atmosphere, the resulting mixture was stirred and heated at 65°C for 3 hours to conduct a reaction. Thereafter, the resulting reaction mixture was cooled to room temperature (25°C).
A yellow solution obtained was filtered, and the filtrate was evaporated to dryness under a vacuum. Yellow residual oil obtained was dissolved in THF (1 mL), hexane (10 mL) was slowly added to the resulting solution to precipitate a yellow solid, and the solid was filtered and dried under vacuum. Thus, Ru catalyst 1 (55 mg, 97%) as yellow crystals was obtained. In the Ru ruthenium catalyst 1 and ligand A shown below, tBu indicates a tertiary butyl group.

[Chem. 23]

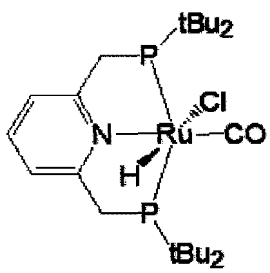

1

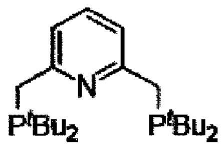

A

$^{31}P\{^1H\}(C_6D_6)$ :90.8 (s), $^1H(C_6D_6)$ :-14.54 (t, 1H, J=20.0Hz), 1.11 (t, 18H, J=8.0Hz), 1.51 (t, 18H, J=8.0Hz), 2.88 (dt, 2H, J=16.0Hz, J=4.0Hz), 3.76 (dt, 2H, J=16.0Hz, J=4.0Hz), 6.45 (d, 2H, J=8.0Hz), 6.79 (t, 1H, J=8.0Hz). $^{13}C\{^1H\}$ NMR$(C_6D_6)$ :29.8 (s), 30.7 (s), 35.2 (t, J=9.5Hz), 37.7 (t, J=6.0Hz), 37.9 (t, J=6.5Hz), 119.5 (t, J=4.5Hz), 136.4 (s), 163.4 (t, J=5.0Hz), 209.8 (s).

[Examples 1 to 6]

1 mL of water was weighed out and put in a glass vial equipped with a stirring rod in a glovebox under an inert gas, 5 mmol of potassium hydrogen carbonate was added, and thereafter a solution obtained by mixing the Ru catalyst 1 in an amount of 0.008 μmol to 0.004 μmol as described in Table 1, 0.008 μmol to 0.044 μmol of the ligand A, and 20 μmol of methyltrioctylammonium chloride with 1 mL of toluene was added. Thereafter, the vial was placed in an autoclave, and the autoclave was sealed and taken out of the glovebox.
The autoclave was heated at 90°C while being stirred. When the temperature reached a target temperature, the autoclave was pressurized to 4.0 MPa with hydrogen. The reaction mixture was stirred for 46 hours, and thereafter the

reaction mixture was cooled with an ice bath, and pressure was carefully released. An upper layer of the solution after the reaction was removed, and an aqueous solution of a lower layer containing potassium formate and unreacted potassium hydrogen carbonate remained. 100 μL of the aqueous solution of the lower layer was collected and dissolved in 500 μL of deuterated water, 300 μL of dimethyl sulfoxide was added as an internal standard, and then potassium formate was quantitively determined by $^1H$ NMR analysis.

[Comparative Example 1]

1 mL of water was weighed out and put in a glass vial equipped with a stirring rod in a glovebox under an inert gas, 5 mmol of potassium hydrogen carbonate was added, and thereafter a solution obtained by mixing 0.004 μmol of the Ru catalyst 1 and 20 μmol of methyltrioctylammonium chloride with 1 mL of toluene was added. Thereafter, the vial was placed in an autoclave, and the autoclave was sealed and taken out of the glovebox.

The autoclave was heated at 90°C while being stirred. When the temperature reached a target temperature, the autoclave was pressurized to 4.0 MPa with hydrogen. The reaction mixture was stirred for 46 hours, and thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 μL of the aqueous solution of the lower layer was collected and dissolved in 500 μL of deuterated water, 300 μL of dimethyl sulfoxide was added as an internal standard, and then potassium formate was quantitively determined by $^1H$ NMR analysis.

[Comparative Example 2]

1 mL of water was weighed out and put in a glass vial equipped with a stirring rod in a glovebox under an inert gas, 5 mmol of potassium hydrogen carbonate was added, and thereafter a solution obtained by mixing 0.004 μmol of the Ru catalyst 1, 0.088 μmol of the ligand A, and 20 μmol of methyltrioctylammonium chloride with 1 mL of toluene was added. Thereafter, the vial was placed in an autoclave, and the autoclave was sealed and taken out of the glovebox.

The autoclave was heated at 90°C while being stirred. When the temperature reached a target temperature, the autoclave was pressurized to 4.0 MPa with hydrogen. The reaction mixture was stirred for 46 hours, and thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 μL of the aqueous solution of the lower layer was collected and dissolved in 500 μL of deuterated water, 300 μL of dimethyl sulfoxide was added as an internal standard, and then potassium formate was quantitively determined by $^1H$ NMR analysis.

[TON Calculation Method]

$$TON = X/Y \quad \text{Formula 1}$$

(In Formula 1, X represents a molar amount X (mol) of the formate calculated by the following Formula 3, and Y represents a molar amount (mol) of the catalyst used in the following reaction.)

[Yield (%) Calculation Method]

$$\text{Yield (\%)} = (X/Z) \times 100 \quad \text{Formula 2}$$

(In Formula 2, X represents a molar amount X (mol) of the formate calculated by the following Formula 3, and Z represents a molar amount (mol) of a carbonate used in the following reaction.)

[Calculation Method for Molar Amount X (mol) of Formate]

$$X = (W/M) \times (Ia \times Ib/R) \times (A/B) \quad \text{Formula 3}$$

(In Formula 3, W represents an amount (g) of dimethyl sulfoxide used to quantify the formate, M represents a molecular weight of dimethyl sulfoxide, R represents a ratio of the number of protons of dimethyl sulfoxide to the number of protons of the formate, Ia represents a proton NMR integration value of the formate, Ib represents a proton NMR integration value of dimethyl sulfoxide, A represents a mass (g) of the aqueous solution of the lower layer obtained by the following reaction, and B represents a mass (g) of the aqueous solution used to quantify the formate.)

[Formate Production]

A carbonate was added to 1 mL of water in a glass vial equipped with a stirring rod in a glovebox under an inert gas, and thereafter the Ru catalyst, the ligand, and 54 μmol of methyltrioctylammonium chloride were added to 1 mL of toluene solvent. Thereafter, the vial was placed in an autoclave, and the autoclave was sealed and taken out of the glovebox.

The autoclave was heated while being stirred. When the temperature reached the target temperature, the autoclave was pressurized with H2. The reaction mixture was stirred, and thereafter the reaction mixture was cooled with an ice bath, and the pressure was carefully released.

An upper layer of the solution after the reaction was removed, and an aqueous solution of a lower layer containing a formate and an unreacted carbonate was obtained.

(Quantification of Formate)

The aqueous solution in the lower layer of B (g) was dissolved in 500 μL of deuterium oxide. Wg of dimethyl sulfoxide was added as an internal standard, and then $^1$H NMR measurement was performed. The NMR integral value of the formate was Ia, and the NMR integral value of dimethyl sulfoxide was Ib.

Examples 1 to 6 and Comparative Examples 1 and 2 are described in Table 1.

[Table 1]

Table 1

| | Example 1 | Example 2 | Example 3 | Example 4 | Example 5 | Example 6 | Comparative Example 1 | Comparative Example 2 |
|---|---|---|---|---|---|---|---|---|
| Amount of Ru catalyst (μmol) | 0.008 | 0.008 | 0.006 | 0.006 | 0.004 | 0.004 | 0.004 | 0.004 |
| Amount of ligand (μmol) | 0.008 | 0.022 | 0.03 | 0.03 | 0.026 | 0.044 | 0 | 0.088 |
| Amount of potassium hydrogen carbonate (mmol) | 5 | 5 | 5 | 5 | 5 | 5 | 5 | 5 |
| Amount of ligand / amount of Ru catalyst | 1 | 2.7 | 5 | 5 | 6.6 | 11 | Without adding ligand | 22 |
| Yield (%) | 18.3 | 64.5 | 79 | 80.5 | 42.9 | 13.4 | 3.72 | 1.86 |
| TON | 151378 | 429173 | 654486 | 667575 | 546814 | 170717 | 47246 | 23623 |

Examples 1 to 6 in which a formate was produced using the production method according to the embodiment show high TON ((Turnover Number) an amount of formate formed (mol) with respect to an amount of catalyst charged (mol)) and are excellent in the production efficiency for a formate. It could be confirmed that by using the ligand in such a manner that the ratio of the substance amount of the ligand to the substance amount of the catalyst in the reaction system

was greater than 0 to 15, a higher TON was obtained compared to Comparative Examples, and the formate could be efficiently formed.

[Protonation of Formate]

In the following Reference Examples 1 and 2, protonation of the formate was carried out using an electrodialyzer (Acilyzer EX3B, manufactured by Astrom).

[Reference Example 1]

A solution obtained by dissolving 165 g of potassium hydroxide in 500 mL of water was placed in the base tank of the electrodialyzer. 500 mL of the aqueous solution containing 5 mol/L of potassium formate was placed in the salt tank. 500 mL of the aqueous solution containing 4.35 mol/L of formic acid was placed in an acid tank. Electrodialysis was performed for 80 minutes at a voltage of 28 V After the dialysis was completed, 100 μL of the solution (acid solution) in the acid tank was collected and dissolved in 500 μL of deuterium oxide, and 300 μL of dimethyl sulfoxide was added as an internal standard, $^1$H NMR was measured to quantitively determine formic acid in the acid solution after the completion of dialysis.

[Reference Example 2]

A solution obtained by dissolving 165 g of potassium hydroxide in 500 mL of water was placed in the base tank of the electrodialyzer. 500 mL of an aqueous solution containing 4.75 mol/L potassium formate and 0.25 mol/L potassium hydrogen carbonate was placed in the salt tank. 500 mL of the aqueous solution containing 4.81 mol/L of formic acid was placed in an acid tank. Electrodialysis was performed for 80 minutes at a voltage of 28 V After the dialysis was completed, 100 μL of the solution (acid solution) in the acid tank was collected and dissolved in 500 μL of deuterium oxide, and 300 μL of dimethyl sulfoxide was added as an internal standard, $^1$H NMR was measured to quantitively determine formic acid in the acid solution after the completion of dialysis.

Reference Examples 1 and 2 are described in Table 2. In Table 2, an initial formate ratio is a percentage of a substance amount X2 relative to a sum of the substance amount (substance amount X2) of the formate and a substance amount of a hydrogen carbonate in the salt tank before the electrodialysis. An initial formate concentration is a molar concentration of the formate in the salt tank before the electrodialysis. An initial hydrogen carbonate concentration is a molar concentration of the hydrogen carbonate in the salt tank before the electrodialysis. An initial formic acid concentration is a molar concentration of formic acid in the acid bath before the electrodialysis. A final formic acid concentration is a molar concentration of formic acid in the acid bath after the completion of electrodialysis. A formic acid yield is a percentage of a substance amount (mol) of formic acid obtained by the electrodialysis relative to the substance amount (substance amount X2) of the formate used in electrodialysis.

[Table 2]

Table 2

| | | Reference Example 1 | Reference Example 2 |
|---|---|---|---|
| Before dialysis | Initial formate ratio (%) | 100 | 95 |
| | Initial formate concentration (mol/L) | 5 | 4.75 |
| | Initial hydrogen carbonate concentration (mol/L) | 0 | 0.25 |
| | Initial formic acid concentration (mol/L) | 4.35 | 4.81 |
| After dialysis | Final formic acid concentration (mol/L) | 6.28 | 6.68 |
| | Formic acid yield (%) | 50.3 | 51.8 |

As can be seen from Table 2, formic acid could be obtained from the formate by electrodialysis. In Reference Example 2 in which the salt tank contained a hydrogen carbonate, formic acid could be obtained in a yield equivalent to that of Reference Example 1 in which the salt tank did not contain a hydrogen carbonate.

INDUSTRIAL APPLICABILITY

The present invention can provide a catalyst reaction method, a method for producing a formate, and a method for producing formic acid that can improve a catalyst efficiency.

While the present invention has been described in detail with reference to specific embodiments, it will be apparent to those skilled in the art that various changes and modifications can be made without departing from the spirit and scope of the present invention.

The present application is based on a Japanese Patent Application (No. 2022-028470) filed on February 25, 2022, the contents of which are incorporated herein by reference.

## Claims

1. A catalyst reaction method, wherein

in a catalyst reaction using at least one catalyst selected from the group consisting of a metal complex represented by the following general formula (1A), a tautomer or stereoisomer of the metal complex, and a salt of the metal complex or the tautomer or stereoisomer, a ligand represented by the following general formula (1B) is used in such a manner that a ratio of a substance amount of the ligand to a substance amount of the catalyst in a reaction system is greater than 0 to 15,

[Chem. 1]

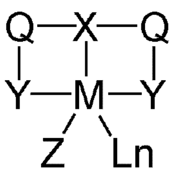

(1A)

(in the general formula (1A), X represents a zero-valent to monovalent atomic group containing a typical element of Groups 13 to 15 and capable of coordinating to M, Q each independently represents a crosslinked structure containing a typical element of Groups 14 to 16 and connecting Y and X, Y each independently represents a zero-valent to monovalent atomic group containing a typical element of Groups 14 to 16 and capable of coordinating to M, M represents a metal atom, Z represents a halogen atom or a hydrogen atom, n represents an integer of 0 to 3, and when more than one L are present, L each independently represents a neutral or anionic ligand), and

[Chem. 2]

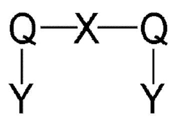

(1B)

(in the general formula (1B), X represents the zero-valent to monovalent atomic group containing the typical element of Groups 13 to 15 and capable of coordinating to M in the general formula (1A), Q each independently represents a crosslinked structure containing a typical element of Groups 14 to 16 and connecting Y and X, and Y each independently represents the zero-valent to monovalent atomic group containing the typical element of Groups 14 to 16 and capable of coordinating to M in the general formula (1A)).

2. The catalyst reaction method according to claim 1, wherein

the metal complex represented by the general formula (1A) is a metal complex represented by the following general formula (2A),

[Chem. 3]

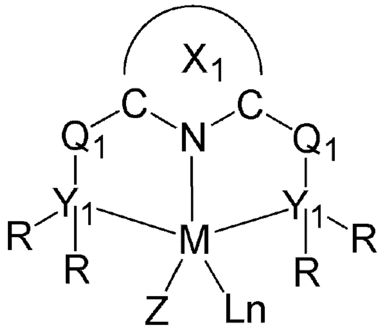

(2A)

(in the general formula (2A), $X_1$ represents a heteroaromatic ring formed with two carbon atoms and a nitrogen atom, which may have a substituent or may be combined with another substituent to form a ring, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand).

**3.** The catalyst reaction method according to claim 2, wherein

the metal complex represented by the general formula (2A) is a metal complex represented by the following general formula (3A),

[Chem. 4]

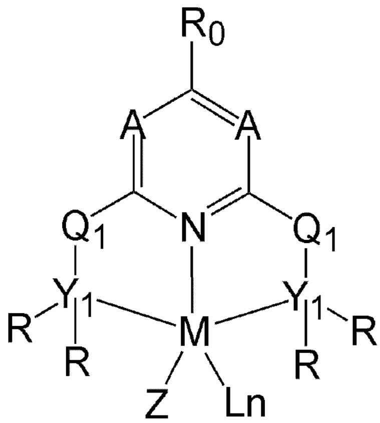

(3A)

(in the general formula (3A), $R_0$ represents a hydrogen atom or an alkyl group, A each independently represents CH, $CR_5$, or N, $R_5$ represents an alkyl group, an aryl group, an aralkyl group, an amino group, a hydroxy group, or an alkoxy group, $Q_1$ each independently represents $CH_2$, NH, or O, $CH_2$ and NH may further have a substituent, $Y_1$ each independently represents a phosphorus atom or a nitrogen atom, R each independently represents an alkyl group, an aryl group, or an aralkyl group, which may further have a substituent, M represents a metal atom, Z represents a halogen atom or a hydrogen atom, n represents an integer of 0 to 3, and when more than one L are present, L each independently represents a neutral or anionic ligand).

**4.** The catalyst reaction method according to claim 1, wherein the metal atom represented by M is ruthenium.

**5.** The catalyst reaction method according to claim 1, wherein the catalyst reaction is an oxidation reaction or a reduction reaction of an organic compound.

**6.** The catalyst reaction method according to claim 1, wherein the catalyst reaction is a formate forming reaction.

**7.** The catalyst reaction method according to claim 6, wherein

the formate forming reaction is a method for producing a formate by allowing hydrogen to react with at least one compound selected from the group consisting of carbon dioxide, a hydrogen carbonate, and a carbonate in the presence of a solvent, and
the formate forming reaction is a two-phase system reaction in which an organic solvent and an aqueous solvent are present in a separated state in the solvent.

**8.** A method for producing a formate, the method comprising:
a first step of producing the formate by the catalyst reaction method according to claim 6 or 7.

**9.** A method for producing formic acid, the method comprising:

a first step of producing a formate by the catalyst reaction method according to claim 6 or 7; and
a second step of protonating at least a part of the formate to form formic acid.

## INTERNATIONAL SEARCH REPORT

International application No.
**PCT/JP2023/006496**

**A. CLASSIFICATION OF SUBJECT MATTER**

***B01J 31/24***(2006.01)i; ***C07B 61/00***(2006.01)i; ***C07C 51/00***(2006.01)i; ***C07C 51/02***(2006.01)i; ***C07C 53/06***(2006.01)i
FI: B01J31/24 Z; C07C51/00; C07C53/06; C07C51/02; C07B61/00 300

According to International Patent Classification (IPC) or to both national classification and IPC

**B. FIELDS SEARCHED**

Minimum documentation searched (classification system followed by classification symbols)

B01J21/00-38/74; C07B61/00; C07C1/00-409/44

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2016-539793 A (KING ABDULLAH UNIVERSITY OF SCIENCE AND TECHNOLOGY) 22 December 2016 (2016-12-22) claims 81, 82, paragraphs [0062]-[0064] | 1-9 |
| Y | JP 9-87224 A (DAICEL CHEM IND LTD) 31 March 1997 (1997-03-31) claim 1, paragraphs [0007], [0014] | 1-9 |
| Y | JP 9-94461 A (DAICEL CHEM IND LTD) 08 April 1997 (1997-04-08) paragraphs [0051]-[0052], table 1 | 1-9 |
| Y | WO 2008/062553 A1 (MITSUI CHEMICALS, INC.) 29 May 2008 (2008-05-29) claims 1, 2, paragraphs [0050], [0060]-[0061] | 1-9 |
| Y | JP 2-91038 A (BP CHEM INTERNATL LTD) 30 March 1990 (1990-03-30) claims, p. 5, lower right column, line 5 to p. 6, upper right column, line 16, examples | 7-9 |
| A | FILONENKO, G. A. et al. Highly efficient reversible hydrogenation of carbon dioxide to formates using a ruthenium PNP-pincer catalyst. ChemCatChem. 2014, vol. 6, pp. 1526-1530 abstract, fig. 1, scheme 1, table 2 | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 April 2023** | **09 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/006496**

**C. DOCUMENTS CONSIDERED TO BE RELEVANT**

| Category* | Citation of document, with indication, where appropriate, of the relevant passages | Relevant to claim No. |
|---|---|---|
| P, X | WO 2022/050234 A1 (NITTO DENKO CORP) 10 March 2022 (2022-03-10) claims, paragraphs [0062]-[0066], examples | 1-9 |

Form PCT/ISA/210 (second sheet) (January 2015)

**INTERNATIONAL SEARCH REPORT**
**Information on patent family members**

International application No.
**PCT/JP2023/006496**

| Patent document cited in search report | Publication date (day/month/year) | Patent family member(s) | Publication date (day/month/year) |
|---|---|---|---|
| JP 2016-539793 A | 22 December 2016 | WO 2015/083007 A1<br>claims 81, 82, paragraphs [0136]-[0137]<br>US 10300469 B1<br>EP 3077109 A1<br>KR 10-2016-0113105 A<br>CN 106413891 A | |
| JP 9-87224 A | 31 March 1997 | (Family: none) | |
| JP 9-94461 A | 08 April 1997 | JP 6-262086 A | |
| WO 2008/062553 A1 | 29 May 2008 | TW 200833640 A | |
| JP 2-91038 A | 30 March 1990 | EP 357243 A2<br>claims, p. 4, lines 25-53, examples | |
| WO 2022/050234 A1 | 10 March 2022 | WO 2022/050235 A1<br>WO 2022/050236 A1<br>TW 202219023 A<br>TW 202219024 A<br>TW 202216277 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 5734286 B
- JP 2013513063 A
- JP 2022028470 A

### Non-patent literature cited in the description

- **E.PIDKO et al.** *ChemCatChem*, 2014, vol. 6, 1526-1530
- **E.PIDKO et al.** *ChemCatChem*, 2014, vol. 6, 1526-1530